# EUROPEAN PATENT SPECIFICATION

(11) **EP 2 480 255 B1**
(45) Date of publication and mention of the grant of the patent: **22.11.2017**
(21) Application number: 10818479.7
(22) Date of filing: 22.09.2010
(51) Int. Cl.: A61K 39/395, A61K 35/66, A61P 1/00

(54) **THERAPY FOR CHRONIC ENTERIC INFECTIONS**
THERAPIE FÜR KRONISCHE DARMINFEKTIONEN
THÉRAPIE POUR INFECTIONS ENTÉRIQUES CHRONIQUES

(30) Priority: 23.09.2009 US 245277 P
(43) Date of publication of application: 01.08.2012
(73) Proprietor: Borody, Thomas Julius, Five Dock, NSW 2046 (AU)
(72) Inventor: Borody, Thomas Julius, Five Dock, NSW 2046 (AU)
(74) Representative: Zwicker, Jörk
(86) International application number: PCT/IB2010/002377
(87) International publication number: WO 2011/036539

(56) References cited:
- EP-A1- 0 955 061
- WO-A1-97/20577
- WO-A1-02/053179
- WO-A2-2005/005481
- US-A1- 2002 037 341
- FULTON R M ET AL: "PREVENTION OF SALMONELLA ENTERITIDIS INFECTION IN COMMERCIAL DUCKLINGS BY ORAL CHICKEN EGG-DERIVED ANTIBODY ALONE OR IN COMBINATION WITH PROBIOTICS", POULTRY SCIENCE, CHAMPAIGN, IL, US, vol. 81, no. 1, 1 January 2002 (2002-01-01), pages 34-40, XP008038267, ISSN: 0032-5791
- MORLEY J E ET AL: "Diarrhea in Long-Term Care: A Messy Problem", JOURNAL OF THE AMERICAN MEDICAL DIRECTORS ASSOCIATION, ELSEVIER, NL, vol. 10, no. 4, 1 May 2009 (2009-05-01), pages 213-217, XP026094020, ISSN: 1525-8610, DOI: 10.1016/J.JAMDA.2009.01.007 [retrieved on 2009-03-28]

## Description

### FIELD OF INVENTION

Non-antibiotic therapy for enteric infections.

### Background of the Invention

The gastrointestinal ("GI") tract is frequently infected by various pathogens. Some transiently infect the bowel flora and mucosa and are removed by the endogenous bacteria or other immune mechanisms (e.g., colonization resistance). Such infections include various strains of Salmonella, Shigella, Campylobacter and various other enteroviruses. There are other enteric infections, however, which are capable of infecting the gastrointestinal tract chronically and result in mild to devastating symptoms and outcomes. Some examples of these pathogens are Clostridium difficile, Clostridium perfringens, Bacillus cereus, Clostridium botulinum, Clostridum tetani, Clostridium welchii, Clostridium sordelli, and various E. coli strains. Still other enteric pathogens lead to the acute infections, which can be overwhelming. Among these pathogens are vibro cholera, Campylobacter jejuni, and Salmonella typhi.

Clostridial infections of the gastrointestinal tract, for example, may result in a group of clinical 'phenotypic' presentations. For example, Clostridium botulinum, may result in several clinical presentations, including fatal botulism due to ingestion of contaminated food, via wound infection, and classically in infant botulism through the colonization of the immature infant flora, between 3 weeks and 11 months after birth, with C botulinum, subsequent toxin production and its entry into the blood with devastating consequences [S Arnon, J Infectious Diseases 1986;154:201]. Sudden Infant Death Syndrome (SIDS) in a similar fashion may be mediated by C botulinum or a closely related pathogen with its entry into the gastrointestinal tract of infants precipitating cardio-respiratory arrest during sleep [S Arnon et al, Lancet 1978;June 17:1273-76;Peterson et Rev. Infect. Dis 1:630 1979].

Another example of these enteric pathogen and their infections is Clostridium tetani which is generally associated with classic neuronal tetanus. Evidence is accumulating, however, that certain strains of Clostridium tetani which enter young children's gastrointestinal tract, e.g., following antibiotic use, may chronically elaborate neurotoxins that are capable of reaching the central nervous system retrograde via the vagus nerve or by the circulation and that result in a clinical condition called "Autism spectrum' [E. Bolte Medical Hypotheses S1. 133-144; 1998]. This includes Autism, Asbergers and Rhett Syndrome as well as ADD and ADHD.

Clostridium perfringens is yet another example of these enteric pathogens. It may be responsible for acute and at times overwhelming diarrhea-predominant gastrointestinal infection-like conditions. Indeed, many patients with chronic "diarrhea-predominant Irritable Bowel Syndrome" [D-IBS] may actually be chronically infected with Clostridium perfringens strains.

Perhaps the most clinically important gastrointestinal infection today is Clostridium difficile infection (CDI). CDI is caused by a gram-positive, spore-forming anaerobic bacillus with a terminal spore. CDI has led to an epidemic in North America with rapidly increasing incidence, severity of disease, and difficulty in treatment. It is the most common hospital acquired infection and may afflict as many as 3,000,000 patients in the US [McFarland et al., New Eng. J. Med., 320:204-10, 1989; Johnson et al., Lancet, 336:97-100, 1990].

Long term use of proton pump inhibitors and treatment of various conditions with antibiotics, and particularly with clindamycin and fluoroquinolones, appear to predispose patients to the development of Clostridium difficile infections.

CDI can result in asymptomatic colonization, mild loose motions or may progress to overwhelming severe diarrhea, pseudomembranous colitis, toxic megacolon, perforation, septicaemia and death. The recent epidemic of the NAP 1 / 027 strain of Clostridium difficile has resulted in a marked increase in morbidity and mortality in North America and Europe. This strain has caused clinically severe disease with markedly increased production of Toxin A and Toxine B, as well as the production of a third toxin, the binary toxin, and has been more resistant to antibiotics. Other important strains of CDI include the 017 and 014 epidemic strains. The CDI epidemic has reached new levels, with about 300 from an estimated 7178 patients present on any one day in U.S. hospitals expected to die as a result of the infections. [W Jarvis, et al. Am J Infection Control 2008;37:263-270]. Therefore, there is an urgent need to develop life-saving and preventative therapies, preferably not relying on antibiotics, the most common root cause of CDI and many other enteric infections, for CDI and the other diseases related to enteric pathogens in animals, including humans.

### SUMMARY OF THE INVENTION

The present invention relates to an antibody or mixture of antibodies directed against an enteric pathogen or group of said pathogens and a probiotic or mixture of probiotics directed against at least some of said pathogens or groups thereof for use in treating chronic enteric pathogen infections in animals suffering from such infections or displaying diseases or conditions consistent with such infections, the antibody component and the probiotic component not being in functional contact until the antibody component has substantially bound the pathogen(s) in the animal.

The present disclosure relates to compositions and methods for the treatment of enteric pathogen infections in animals, including humans, suffering from such infections or displaying diseases or conditions consistent with such infections.

In another embodiment, the present disclosure relates to compositions and methods to prevent, or to at least reduce the likelihood of enteric pathogen infections in animals, including humans, that are at risk of such infections.

The present disclosure in another embodiment relates to methods that comprise the serial or separated delivery of antibodies directed against enteric pathogens, to animals, including humans, suffering from infections related to such pathogens or displaying diseases or conditions consistent with such infections, or at risk of developing these infections, followed by the delivery to those subjects, of probiotics directed against at least some of such pathogens. These methods treat, prevent or reduce the acute and chronic infections and infestations of the gastrointestinal tract in humans and other animals by enteric pathogens.

In another embodiment, the present disclosure relates to compositions that allow the serial or separated delivery to animals, including humans, suffering from infections caused by enteric pathogens or displaying diseases or conditions consistent with such infections, or at risk of developing such infections of antibodies directed against enteric pathogens and then the delivery of probiotics directed against the same pathogens.

In another embodiment, the present disclosure relates to methods of making the compositions and using the methods of this disclosure.

In another embodiment, the present disclosure relates to methods and compositions that treat, prevent or reduce clinical conditions or diseases which may be related to enteric pathogen infections but which causative pathogens are not known, e.g., IBS or travelers diarrhea. In spite of the absence of identifiable pathogens in such infections, the methods and compositions of this disclosure still have a positive effect on the various enteric pathogen infections driving these illnesses.

### DETAILED DESCRIPTION OF THE INVENTION

The present invention relates to an antibody or mixture of antibodies directed against an enteric pathogen or group of said pathogens and a probiotic or mixture of probiotics directed against at least some of said pathogens or groups thereof for use in treating chronic enteric pathogen infections in animals suffering from such infections or displaying diseases or conditions consistent with such infections, the antibody component and the probiotic component not being in functional contact until the antibody component has substantially bound the pathogen(s) in the animal.

Disclosed herein are methods and compositions for the treatment and prophylaxis of enteric pathogen infections, diseases and conditions in animals, including humans.

The methods of the present disclosure comprise the step of administering, serially or separately, (1) antibodies directed against a pathogen or group of pathogens that are related to enteric infections and (2) probiotics directed against at least some of said pathogen or group of pathogens to humans or other animals suffering from infections by the pathogens, or displaying diseases or conditions consistent with such infections, or at risk of developing such infections.

The compositions of the present disclosure comprise antibodies directed against a pathogen or group of pathogens that are related to enteric infections and probiotics directed against at least some of those pathogens. The compositions of the present disclosure are formulated or administered so as to prevent the antibodies and the probiotics coming into functional contact with each other before the antibodies have substantially bound to the pathogen(s) in the human or other animal.

As used herein "enteric pathogens" or pathogens that are "related to or consistent with enteric infections" refer to organisms capable of causing an infection in the gastrointestinal tract of an animal, including a human.

Examples of enteric pathogenic organisms include and are not limited to Aeromonas hydrophilia, Bacillus cereus, Vibrio parahemolyticus, Vibrio cholerae 01, Vibrio cholera non-01, Vibrio vulnificus, Salmonella enteric, Salmonella typhi, Salmonella paratyphi, Salmonella entertidis, Salmonella cholerasuis, Salmonella typhimurium, Clostridium difficile, Clostridium botulinum, Clostridium perfringens, Staphylococcus aureus, Escherichia coli (- various subclasses), Campylobacter jejuni, Campylobacter coli, Campylobacter lari, Campylobacter fetus, Yersinia enterocolitica, Yersinia pestis, Yersinia pseudotuberculosis, Plesiomonas shigelloides, Listeria monocytogenes, enteric viruses, for example, rotavirus, Norwalk-like viruses, enteric adenoviruses, coronavirus and all other non-enveloped enteroviruses, and enteric parasites and fungi, for example, Cryptosporidium, and Cyclospora, luminal and tissue mycobacteria, such as Mycobacterium avium avium, Mycobacterium avium paratuberculosis, Mycobacterium avium silvaticum and other components of the Mycobacterium avium complex, Helicobacter pylori, Giardia lamblia and other parasites including Dientamoeba fragilis, Blastocystis hominis and Entamoeba histolytica.

Among the diseases and infections, without limitation, that are related to or consistent with enteric pathogens are Irritable Bowel Syndrome in its various forms (diarrhea, pain, constipation, predominant or mixtures thereof), bloating, small bowel bacterial overgrowth, diverticular disease, including diverticulitis, colitis (ulcerative, Crohn's, lymphocytic, microscopic, indeterminate pseudo membranous, proctitis, post infective colitis) among others, as well as Crohn's Disease, idiopathic ileitis, constipation, flatulence, and halitosis, dysmotility conditions, including gastronaresis, reflux disease, pseudo-obstruction, bloating and traveler's diarrhea, as well as Parkinson's disease constipation.

As described herein, the present disclosure in one embodiment comprises a pharmaceutical composition comprising antibodies directed against an enteric pathogen or group of enteric pathogens and a probiotic or group of probiotics that are likewise directed against at least some of those pathogens. As used herein, the composition may have the two components, the antibody component and the probiotic component, together in one delivery unit. In this embodiment, the two components are functionally separated in the delivery unit (e.g. by coating of capsules or microencapsulation). For example, the probiotic component may be encapsulated, such that it is delivered to the human or other animal, more slowly or later than the antibody component of the composition. Alternatively, the antibody component comprises the outside or outer layer of a capsule or other delivery unit or is coated on a capsule and the probiotic component comprises the inner part of the capsule. In either alternative, the probiotic component is delivered to the human or other animal after the antibody component has substantially bound to the pathogen(s).

A composition as used herein may also have the antibody component and the probiotic component in separate delivery systems or units. For example, two separate capsules, sachets, tablets, granules or pills. Again, the intent is to deliver the probiotic component after the antibody component has substantially bound to the pathogen(s).

The compositions of the present disclosure can also comprise suppositories, enemas, or can be made into suspensions to be infused transendoscopically or trans-colonoscopically into the duodenum, terminal ileum or via an enteric tube into the jejunum. For example, in patients whose access to the GI tract has to be obtained in intensive care situations. The composition may also be administered combined with drinks or foods to be ingested serially, e.g., morning ingestion of the antibody component followed, preferably 4-12 hrs later, by the probiotic component of the composition.

The probiotic component of the composition of the present disclosure is a micro organism selected, for example, from the group consisting of Lactobacilli, Bifidobacteria, E coli, Eubacteria, Saccharomyces species, Enterococci, Bacteroides or non pathogenic Clostridia, e.g. Clostridium butyricum and nonpathogenic C difficile. As will be appreciated by one of skilled in the art, other suitable probiotics known in the art may also be used in the compositions of the present disclosure.

As used in this disclosure, the probiotic component is one or a group of probiotic organisms that are directed at the pathogen or group of pathogens being targeted or whose risk of infections is being reduced or prevented. As used herein, a probiotic(s) that is directed to an enteric pathogen or group of those pathogens are those probiotics that are capable in culture of eradicating or suppressing the growth of the targeted pathogen or pathogens. Thus, the probiotics of the compositions of this the present disclosure are preferably selected by co-culturing the enteric pathogens with a probiotic or groups thereof and selecting the probiotics or group that inhibits or suppresses growth of at least some of the pathogen(s).

The antibody component of the composition of the present disclosure, may be polyclonal antibodies, monoclonal antibodies, Fab, Fab', F(ab') .sub.2, Fv, dAb, and complementarity determining region (CDR) fragments, single-chain antibodies (scFv)., chimeric antibodies, humanized or human antibodies, diabodies and polypeptides that contain at least a portion of an immunoglobulin that is sufficient to confer specific antigen binding. Preferably, they are polyclonal antibodies derived from eggs, including egg yolk and albumin, from poultry immunized with at least one antigen derived from at least one enteric pathogen. When the antibodies are monoclonal antibodies, they are also preferably mixtures of monoclonal antibodies or mixtures of monoclonal antibodies and polyclonal antibodies.

The antibodies of the compositions of the present disclosure are directed against antigens of the enteric pathogens being targeted. The antigens may comprise whole organisms, spores, fimbriae, pilli capsules, glycocalyces, secreted enzymes, e.g. collagenase, hyaluronidase, coagulase, protease, immunoglobin, proteins isolated from cell membrane, lipopolysaccharide fraction as well as attenuated virus particles, toxins, viral proteins and cell surface proteins as well as fragments and mixtures thereof. Preferably, mixtures are used.

For example, it is particularly preferred to use as the antigen multiple strains of the pathogen(s) being targeted, or fragments or products of those strains. In one embodiment, for example, strains 027, 017, and 014 of CDI and fragments and toxins produced by those strains are used.

The antibody components of the compositions of the present disclosure are preferably produced by immunizing a host, preferably poultry and most preferably chickens, with a diverse collection of antigens or immunogens or a group of antigens and immunogens derived from a group of pathogens. However, other hosts known in the art for antibody production including sheep, horses, and cows, can be used. Preferably, IgY polyclonal antibodies are used in the methods and compositions of the present disclosure.

An alternate approach of antibody generation is the production of monoclonal antibodies. Again, however, it is preferred to use diverse mixtures of monoclonal antibodies in the methods and compositions of the present disclosure. For example, when treating CDI, a mixture of monoclonal antibodies directed to Toxin A, Toxin B. or the binary toxin, or preferably all these are used. In the more preferred embodiments of a composition for treating CDI, the antibody component would contain a multiplicity of monoclonal antibodies, selected from the groups of antibodies directed to Toxin A, Toxin B, binary Toxin and supernatant toxins yet to be identified but able to be used to immunize from the supernantant, vegetative forms of the bacterium fimbriae, glycocalyces, pilli, spores, capsules, secreted enzymes e.g. collagenase, hyaluronidase, coagulase and immunoglobulin A protease, proteins and lipids isolated from the cell membranes and the lipopolysaccharide fraction spore and fractions of spores.

Monoclonal antibodies can be used alone (singularly) or preferably in combinations (mixtures) and alone or with polyclonal antibodies.

In a preferred embodiment when antibodies raised in chickens are used, the egg product, egg yolk alone or the whole egg content may be used to produce an antibody powder for administration after e.g. freeze drying spray drying. The whole egg versus yolk alone is preferred because of the increased amount of IgY available and the presence of the albumin as a "support" vehicle to stimulate the antibody-antigen binding within the GI tract during administration.

The amount of the antibody component and the probiotic component and the other treatment parameters of the compositions of the present disclosure and in the methods of the present disclosure are easily determined by those of skill in the art taking into account the patient, his or her history, the infection or condition being treated and the effect of various treatments.

The methods and compositions of the present disclosure may also include additional steps or components. For example, in one embodiment, the human or other animal is pre-treated with antibiotics targeted against the enteric pathogens to reduce the infective load or numbers of the pathogens in the enteric tract. For example, in the situation where a patient is infected with Clostridium difficile pre-treatment with anti-clostridial agents including metronidazole, vancomycin, rifampicin, rifaximin, nitazoxanide or rifabutin used singly or in combinations, for at least one day and up to 3 months - reduces the load of the bacteria and spores.

In a further embodiment of the present disclosure, the effectiveness of the methods and compositions may be enhanced by reducing the acid in the stomach during antibody ingestion to prevent acid damage of the antibody protein [curdling] . This can be achieved with H2 receptor antagonists, but more preferably by proton pump inhibitors - PPI's (e.g. omeprazole, lansoprazole, esomeprazole, pantoprazole, rabeprazole and other PPI's), administered prior to the ingestion of the medications. Ideally, the PPI should be given at least 2-4 hours before and in some situations a PPI could be combined with a H2RA to maximize acid suppression. This will allow the passage both of the probiotic component and especially the antibody component of the compositions of this disclosure through the gastric space without causing precipitation of the large proteins which make up the antibodies.

In some embodiments of the present disclosure species other than humans benefit from methods compositions of this disclosure. In particular, for example, dogs which develop chronic Clostridium perfringens diarrhoea and other such diarrhea conditions caused by specific and non-specific pathogens may be treated in accordance with this disclosure.

In the prophylactic embodiment of the present disclosure, the compositions of the present disclosure are administered, often at lower doses than in the situation of active infection, to humans and other animals at risk of enteric pathogen infections.

### Examples

### Example 1

A 48 yr old female with longstanding and recurrent urine infections treated with antibiotics developed chronic diarrhoea. After a number of stool tests toxigenic Clostridium difficile was detected in the stool. This was a non-epidemic strain but nevertheless caused chronic diarrhoea which occurred between 10 and 15 times per day causing occasional incontinence.

The patient had been treated initially with 20 gm/d of C difficile immune egg powder preparation for 10 days but her stool continued to be C difficile-positive and her diarrhoea recurred. She was then given a combination of 10 gm of the same egg powder but this time together with Lactobacillus rhamnosus strain CDD1. This strain was selected because it could inhibit C. difficile in vitro and was added at a dose which was equivalent to 10¹⁰ bacteria for ten days. The antibodies were administered in the morning and the probiotic bacteria eight hours later for 10 days.

On completion of the study (at 4 and 8 weeks), the patient was free of C. difficile infection on stool tests. Her diarrhoea settled by day 3 of the combined therapy.

### Example 2

A 9 year old male allergic to penicillin was given prophylactic "clindamycin" following a cut finger which was then sutured in the emergency room in a San Francisco hospital. 3 to 4 weeks after finishing the clindamycin he developed diarrhoea. This diarrhoea was associated with cramping, urgency, malaise and progressive weight loss of about 2 to 3 kg. He was diagnosed as having the epidemic strain of C. difficile and was given metronidazole. He developed nausea and was then given vancomycin capsules 250 mg tds. His diarrhoea was inhibited quite effectively both by the metronidazole and by the vancomycin. Within 2 to 4 weeks of stopping the medications the diarrhoea would reccur with up to 8 or 12 diarrhoeal stools per day. Over the next 18 months there were numerous recurrences of the diarrhoea each time suppressed by vancomycin. Numerous protocols of reducing doses of Vancomycin were tried but he continued to have diarrhoea.

The patient was then treated with 10 gm daily of the anti-C. difficile antibodies for 10 days. His diarrhoea initially settled but then recurred 3 to 4 weeks after the cessation of the 10 day treatment. He was then given a 10 gm morning dose of the antibody followed by an evening dose of a Bifidobacterium strain CDD2 strain that could inhibit C. difficile as a combination therapy for 10 days. The diarrhoea again ceased by day 3 and has not recurred now for nearly 8 months. His stool remain C difficile negative. The patient has gained weight, has formed stools, has no pain, no urgency and no incontinence.

## Claims

1. An antibody or mixture of antibodies directed against an enteric pathogen or group of said pathogens and a probiotic or mixture of probiotics directed against at least some of said pathogens or groups thereof for use in treating chronic enteric pathogen infections in animals suffering from such infections or displaying diseases or conditions consistent with such infections, the antibody component and the probiotic component not being in functional contact until the antibody component has substantially bound the pathogen(s) in the animal.

2. A composition comprising an antibody or mixture of antibodies directed against an enteric pathogen or group of said pathogens and a probiotic or mixture of probiotics directed against at least some of said pathogens or groups thereof, for use in treating chronic enteric pathogen infections in animals suffering from such infections or displaying diseases or conditions consistent with such infections, the antibody component not being in functional contact with the probiotic component such that on delivery of the composition the antibody component substantially binds to the pathogen prior to delivery of the probiotic.

3. The composition for use according to claim 2 comprising the two components functionally separated in a delivery unit.

4. The combination for use according to claim 1 wherein the animal is a human.

5. The combination or composition for use according to any of claims 1 to 4 wherein the pathogen or group of pathogens are selected from the group consisting of Aeromonas hydrophilia, Bacillus cereus, Vibrio parahemolyticus, Vibrio cholerae 01, Vibrio cholera non-01, Vibrio vulnificus, Salmonella enteric, Salmonella typhi, Salmonella paratyphi, Salmonella entertidis, Salmonella cholerasuis, Salmonella typhimurium, Clostridium difficile, Clostridium botulinum, Clostridium perfringens, Staphylococcus aureus, Escherichia coli, Campylobacter jejuni, Campylobacter coli, Campylobacter lari, Campylobacter fetus, Yersinia enterocolitica, Yersinia pestis, Yersinia pseudotuberculosis, Plesiomonas shigelloides, Listeria monocytogenes, enteric viruses, parasites and fungi, luminal and tissue mycobacteria, Helicobacter pylori, Giardia lamblia, Dientamoeba fragilis, Blastocystis hominis and Entamoeba histolytica; or wherein the pathogen or group of pathogens is selected from pathogens related to CDI and fragments, components, and products of those pathogens.

6. The combination or composition for use according to claim 5 wherein the enteric viruses are selected from rotavirus, Norwalk-like viruses, enteric adenoviruses, coronavirus and other non-enveloped enteroviruses, wherein the fungi is selected from Cryptosporidium and Cyclospora, and wherein the mycobacteria is selected from Mycobacterium avium avium, Mycobacterium avium paratuberculosis, Mycobacterium avium silvaticum and other components of the Mycobacterium avium complex.

7. The combination or composition for use according to any one of claims 1 to 6 wherein the antibody or group of antibodies are selected from polyclonal antibodies, monoclonal antibodies, mixtures of polyclonal antibodies and monoclonal antibodies, Fab, Fab', F(ab') sub.2, Fv, dAb, and complementarity determining region (CDR) fragments, single-chain antibodies (scFv), chimeric antibodies, humanized or human antibodies, diabodies and polypeptides that contain at least a portion of
an immunoglobulin that is sufficient to confer specific antigen binding or wherein the antibodies are selected from the groups of antibodies directed to Toxin A, Toxin B, binary Toxin and other supernatant toxins able to be used to immunize from the supernatant, vegetative forms of the bacterium fimbriae, glycocalyces, pilli, spores, capsules, secreted enzymes, proteins and lipids isolated from the cell membranes and the lipopolysaccharide fraction spore and fractions of spores, or
wherein the antibody or group of antibodies are IgY antibodies raised in chickens.

8. The combination or the composition for use according to claim 7, wherein the secreted enzymes are selected from collagenase, hyaluronidase, coagulase and immunoglobulin A protease.

9. The combination or composition for use according to any one of claims 1 to 8, wherein the probiotic or mixture of probiotics are selected from the group consisting of Lactobacilli, Bifidobacteria, E. coli, Eubacteria, Saccharomyces, Enterococci, Bacteroides and non pathogenic Clostridia.

10. The combination or composition for use according to claim 9, wherein the non pathogenic Clostridia is selected from Clostridium butyricum and nonpathogenic C. difficile.

11. The combination or composition for use according to a ny one of claims 1 to 10 wherein the probiotic or mixture of probiotics are capable of eradicating or suppressing the growth of said pathogen(s) in vitro or in vivo.

12. The combination or composition for use according to any one of claims 1 to 11 wherein the infections are selected from the group consisting of Irritable Bowel Syndrome, bloating, small bowel bacterial overgrowth, diverticular disease, colitis, Crohn's Disease, idiopathic ileitis, constipation, flatulence, and halitosis, dysmotility conditions, reflux disease, pseudo-obstruction, bloating and traveler's diarrhea and Parkinson's disease constipation.

13. The combination or composition for use according to claim 12, wherein the Irritable bowel syndrome is selected from diarrhea, pain, constipation, predominant and mixtures thereof, wherein the diverticular disease is diverticulitis, wherein colitis is selected from ulcerative, Crohn's lymphocytic, microscopic, indeterminate pseudo membranous, proctitis and post infective colitis, and wherein the dysmotility condition is gastroparesis.

14. The combination or composition for use according to any one of claims 1 to 13 wherein the antibody component and the probiotic component are contained in separate delivery systems or units.

15. The combination or composition for use according to claim 14, wherein the delivery systems or units are in form of capsules, sachets, tablets, granules, pills, suppositories, enemas, or suspensions capable of being infused trans-endoscopically or trans-colonoscopically into the duodenum, terminal ileum or via an enteric tube into the jejunum.

## Patentansprüche

1. Ein Antikörper oder eine Mischung von Antikörpern, der/die gegen ein enterales Pathogen oder eine Gruppe dieser Pathogene gerichtet ist/sind und ein Probiotikum oder eine Mischung von Probiotika, das/die gegen zumindest einige dieser Pathogene oder Gruppen davon gerichtet ist/sind, zur Verwendung bei der Behandlung von chronischen enteralen Pathogen-Infektionen bei Tieren, die an solchen Infektionen leiden oder Krankheiten oder Zustände zeigen, die mit solchen Infektionen im Einklang stehen, wobei die Antikörper- Komponente und die Probiotikum-Komponente nicht in funktionellem Kontakt stehen, bis die Antikörper-Komponente das/die Pathogen(e) in dem Tier in wesentlichem Ausmaß gebunden hat.

2. Eine Zusammensetzung umfassend einen Antikörper oder eine Mischung von Antikörpern, der/die gegen ein enterales Pathogen oder eine Gruppe dieser Pathogene gerichtet ist/sind und ein Probiotikum oder eine Mischung von Probiotika, das/die gegen zumindest einige dieser Pathogene oder Gruppen davon gerichtet ist/sind, zur Verwendung bei der Behandlung von chronischen enteralen Pathogen-Infektionen bei Tieren, die an solchen Infektionen leiden oder Krankheiten oder Zustände zeigen, die mit solchen Infektionen im Einklang stehen, wobei die Antikörper-Komponente und die Probiotikum-Komponente nicht in funktionellem Kontakt stehen, so dass bei der Abgabe der Zusammensetzung die Antikörper-Komponente das Pathogen in wesentlichem Ausmaß vor der Abgabe des Probiotikums bindet.

3. Zusammensetzung zur Verwendung nach Anspruch 2, welche die beiden Komponenten funktionell voneinander getrennt in einer Abgabeeinheit umfasst.

4. Kombination zur Verwendung nach Anspruch 1, wobei das Tier ein Mensch ist.

5. Kombination oder Zusammensetzung zur Verwendung nach einem der Ansprüche 1 bis 4 wobei das Pathogen oder die Gruppe von Pathogenen ausgewählt sind aus der Gruppe bestehend aus Aeromonas hydrophilia, Bacillus cereus, Vibrio parahemolyticus, Vibrio cholerae 01, Vibrio cholera non-01, Vibrio vulnificus, Salmonella enteric, Salmonella typhi, Salmonella paratyphi, Salmonella entertidis, Salmonella cholerasuis, Salmonella typhimurium, Clostridium difficile, Clostridium botulinum, Clostridium perfringens, Staphylococcus aureus, Escherichia coli, Campylobacter jejuni, Campylobacter coli, Campylobacter lari, Campylobacter fetus, Yersinia enterocolitica, Yersinia pestis, Yersinia pseudotuberculosis, Plesiomonas shigelloides, Listeria monocytogenes, enteralen Viren, Parasiten und Pilzen, luminale Mykobakterien und Gewebe-Mykobakterien, Helicobacter pylori, Giardia lamblia, Dientamoeba fragilis, Blastocystis hominis und Entamoeba histolytica; oder wobei das Pathogen oder die Gruppe von Pathogenen ausgewählt ist aus Pathogenen die mit CDI in Zusammenhang stehen, und Fragmenten, Komponenten und Produkten dieser Pathogene.

6. Kombination oder Zusammensetzung zur Verwendung nach Anspruch 5, wobei die enteralen Viren ausgewählt sind aus Rotavirus, Norwalk-ähnlichen Viren, enteralen Adenoviren, Coronavirus und anderen nicht-umhüllten Enteroviren, wobei die Pilze ausgewählt sind aus Cryptosporidium und Cyclospora, und wobei die Mykobakterien ausgewählt sind aus Mycobacterium avium avium, Mycobacterium avium paratuberculosis, Mycobacterium avium silvaticum und anderen Bestandteilen des Mycobacterium avium-Komplexes.

7. Kombination oder Zusammensetzung zur Verwendung nach einem der Ansprüche 1 bis 6 wobei der Antikörper oder die Gruppe von Antikörpern ausgewählt sind aus polyklonalen Antikörpern, monoklonalen Antikörpern, Mischungen von polyklonalen Antikörpern und monoklonalen Antikörpern, Fab, Fab', F(ab') sub.2, Fv, dAb und Complementary Determining Region (CDR)-Fragmenten, Einzelketten-Antikörpern (scFv), chimären Antikörpern, humanisierten oder menschlichen Antikörpern, Diabodies und Polypeptiden, die mindestens einen Teil von einem Immunglobulin enthalten, der ausreicht, um eine spezifische Antigenbindung zu gewähren oder wobei die Antikörper aus den Gruppen von Antikörpern ausgewählt sind, die gerichtet sind auf Toxin A, Toxin B, binäres Toxin und andere Überstand-Toxine, die verwendet werden können, um aus dem Überstand zu immunisieren, vegetative Formen der Bakterien-Fimbriae, Glycocalyces, Pilli, Sporen, Kapseln, sezernierten Enzyme, Proteine und Lipide, die aus den Zellmembranen isoliert werden, und der Lipopolysaccharidfraktion von Sporen und Fraktionen von Sporen, oder
wobei der Antikörper oder die Gruppe von Antikörpern in Hühnern gezogene IgY-Antikörper sind.

8. Kombination oder Zusammensetzung zur Verwendung nach Anspruch 7, wobei die sezernierten Enzyme ausgewählt sind aus Kollagenase, Hyaluronidase, Koagulase und Immunglobulin-A-Protease.

9. Kombination oder Zusammensetzung zur Verwendung nach einem der Ansprüche 1 bis 8, wobei das Probiotikum oder die Mischung von Probiotika ausgewählt sind aus der Gruppe bestehend aus Lactobacilli, Bifidobacteria, E. coli, Eubacteria, Saccharomyces, Enterokokken, Bacteroides und nicht pathogene Clostridia.

10. Kombination oder Zusammensetzung zur Verwendung nach Anspruch 9, wobei die nicht
pathogenen Clostridia ausgewählt sind aus Clostridium butyricum und nichtpathogenen C. difficile.

11. Kombination oder Zusammensetzung zur Verwendung nach einem der Ansprüche 1 bis 10
wobei das Probiotikum oder die Mischung von Probiotika das Wachstum des besagten Pathogens oder der besagten Pathogene in vitro oder in vivo unterbinden oder unterdrücken können.

12. Kombination oder Zusammensetzung zur Verwendung nach einem der Ansprüche 1 bis 11, wobei die Infektionen ausgewählt sind aus der Gruppe bestehend aus Reizdarm Syndrom, Aufgedunsenheit, Dünndarmfehlbesiedlung, Divertikelkrankheit, Colitis, Morbus Crohn, idiopathische Ileitis, Verstopfung, Blähungen, und Mundgeruch, Dysmotilitäts-Zustände, Reflux-Krankheit, Pseudoobstruktion, Blähungen und Reisedurchfall und Verstopfung bei Parkinson-Erkrankung.

13. Kombination oder Zusammensetzung zur Verwendung nach Anspruch 12, wobei das Reizdarmsyndrom ausgewählt ist aus Durchfall, Schmerz, Verstopfung, vorherrschend und Mischungen davon, wobei die Divertikelkrankheit Divertikulitis ist, wobei die Colitis ausgewählt ist aus ulzerativ, Crohn's lymphozytisch, mikroskopisch, unbestimmt pseudomembranös, Proktitis und postinfektiöser Colitis, und wobei der Dysmotilitäts-Zustand Gastroparese ist.

14. Kombination oder Zusammensetzung zur Verwendung nach einem der Ansprüche 1 bis 13, wobei die Antikörperkomponente und die probiotische Komponente in getrennten Abgabesystemen oder -einheiten enthalten sind.

15. Kombination oder Zusammensetzung zur Verwendung nach Anspruch 14, wobei die Abgabesysteme oder -einheiten in Form von Kapseln, Beuteln, Tabletten, Granulaten, Pillen, Suppositorien, Klistieren oder Suspensionen bestehen, welche transendoskopisch oder trans-koloskopisch in den Zwölffingerdarm, das terminale Ileum oder mittels einer enteralen Sonde in das Jejunum infundiert werden können.

## Revendications

1. Anticorps ou mélange d'anticorps dirigés contre un agent pathogène entérique ou un groupe desdits agents pathogènes et agent probiotique ou mélange d'agents probiotiques dirigés contre au moins certains desdits agents pathogènes ou groupes de ceux-ci pour utilisation dans le traitement d'infections pathogènes entériques chroniques chez des animaux souffrant de telles infections ou présentant des affections ou des états en accord avec de telles infections, le composant anticorps et le composant probiotique n'étant pas en contact fonctionnel jusqu'à ce que le composant anticorps ait substantiellement fixé le ou les agent(s) pathogène(e) chez l'animal.

2. Composition comprenant un anticorps ou mélange d'anticorps dirigés contre un agent pathogène entérique ou un groupe desdits agents pathogènes et un agent probiotique ou mélange d'agents probiotiques dirigés contre au moins certains desdits agents pathogènes ou groupes de ceux-ci pour utilisation dans le traitement d'infections pathogènes entériques chroniques chez des animaux souffrant de telles infections ou présentant des affections ou des états conformes à de telles infections, tandis que le composant anticorps n'est pas en contact fonctionnel avec le composant probiotique, de telle sorte que lors de la libération de la composition le composant anticorps se lie substantiellement à l'agent pathogène avant la libération de l'agent probiotique.

3. Composition pour utilisation selon la revendication 2 comprenant les deux composants fonctionnellement séparés dans une unité de libération.

4. Combinaison pour utilisation selon la revendication 1 dans laquelle l'animal est un humain.

5. Combinaison ou composition pour utilisation selon l'une quelconque des revendications 1 à 4 où l'agent pathogène ou le groupe d'agents pathogènes sont choisis dans le groupe consistant en Aeromonas hydrophilia, Bacillus cereus, Vibrio parahemolyticus, Vibrio cholerae 01, Vibrio cholerea non-01, Vibrio vulnificus, Salmonella enteric, Salmonella typhi, Salmonella paratyphi, Salmonella entertidis, Salmonella cholerasuis, Salmonella typhimurium, Clostridium difficile, Clostridium botulinum, Clostridium perfringens, Staphylococcus aureus, Escherichia coli, Campylobacter jejuni, Campylobacter coli, Campylobacter lari, Campylobacter fetus, Yersinia enterocolitica, Yersinia pestis, Yersinia pseudotuberculosis, Plesiomonas shigelloides, Listeria monocytogenes, virus entériques, parasites et champignons, mycobactéries luminales et tissulaires, Helicobacter pylori, Giardia lamblia, Dientamoeba fragilis, Blastocystis hominis et Entamoeba histolytica; ou dans laquelle l'agent pathogène ou le groupe d'agents pathogènes est choisi parmi des agents pathogènes apparentés à CDI et des fragments, composants, et produits de ces agents pathogènes.

6. Combinaison ou composition pour utilisation selon la revendication 5 où les virus entériques sont choisis parmi rétrovirus, virus analogues au Norwalk, adénovirus entériques, coronavirus et autres entérovirus sans enveloppe, tandis que le champignon est choisi parmi Cryptosporidium et Cyclospora, et où la mycobactérie est choisie parmi Mycobacterium avium avium, Mycobacterium avium paratuberculosis, Mycobacterium avium silvaticum et autres composants du complexe de Mycobacterium avium.

7. Combinaison ou composition pour utilisation selon l'une quelconque des revendications 1 à 6 où l'anticorps ou le groupe d'anticorps sont choisis parmi les anticorps polyclonaux, les anticorps monoclonaux, des mélanges d'anticorps polyclonaux et d'anticorps monoclonaux, Fab, Fab', F(ab') sub. 2, Fv, dAb, et des fragments de régions de détermination de complémentarité (CDR), des anticorps monochaîne (scFv), des anticorps chimériques, des anticorps humanisés ou humains, des diacorps et des polypeptides qui contiennent au moins une portion d'une immunoglobuline qui est suffisante pour conférer une liaison à l'antigène spécifique ou
dans laquelle les anticorps sont choisis parmi les groupes d'anticorps dirigés contre la Toxine A, la Toxine B, la Toxine binaire et d'autres toxines surnageantes aptes à être utilisées pour l'immunisation à partir du surnageant, de formes végétatives du Bacterium fimbriae, de glycocalyces, de pilli, de spores, de capsules, d'enzymes sécrétées, de protéines et lipides isolés à partir des membranes cellulaires, et de la fraction lipopolysaccharide du spore et des fractions de spores, ou
dans laquelle l'anticorps ou le groupe d'anticorps sont des anticorps IgY élevés sur des poulets.

8. Combinaison ou composition pour utilisation selon la revendication 7, où les enzymes sécrétées sont choisies parmi collagénase, hyaluronidase, coagulase et protéase d'immunoglobuline A.

9. Combinaison ou composition pour utilisation selon l'une quelconque des revendications 1 à 8, où l'agent probiotique ou le mélange d'agents probiotiques sont choisis dans le groupe consistant en Lactobacilli, Bifidobacteria, E. coli, Eubacteria, Saccharomyces, Enterococci, Bactéroïdes et Clostridia non pathogène.

10. Combinaison ou composition pour utilisation selon la revendication 9, où le Clostridia non pathogène est choisi parmi Clostridium butyricum et C. difficile non-pathogène.

11. Combinaison ou composition pour utilisation selon l'une quelconque des revendications 1 à 10 où l'agent probiotique ou le mélange d'agents probiotiques sont capables d'éradiquer ou de supprimer la croissance dudit(desdits) agent(s) pathogène(s) in vitro ou in vivo.

12. Combinaison ou composition pour utilisation selon l'une quelconque des revendications 1 à 11 où les infections sont choisies dans le groupe consistant en syndrome du côlon irritable, ballonnement, prolifération bactérienne intestinale, affection diverticulaire, colite, maladie de Crohn, iléite idiopathique, constipation, flatulence, et haleine fétide, états de dysmotilité, affection de reflux, pseudo-obstruction, ballonnement et diarrhée du voyageur ainsi que constipation dans la maladie de Parkinson.

13. Combinaison ou composition pour utilisation selon la revendication 12, où le syndrome du côlon irritable est choisi parmi diarrhée, douleur, constipation, prédominantes et leurs mélanges, tandis que l'affection diverticulaire est la diverticulite, tandis que la colite est choisie parmi les colites ulcérative, lymphocytique de Crohn, microscopique, pseudo membranaire indéterminée, proctite et colite post infectieuse, et où l'état de dysmotilité est la gastroparésie.

14. Combinaison ou composition pour utilisation selon l'une quelconque des revendications 1 à 13 où le composant anticorps et le composant probiotique sont contenus dans des systèmes ou des unités de libération séparés.

15. Combinaison ou composition pour utilisation selon la revendication 14, où les systèmes ou unités de libération sont sous la forme de capsules, sachets, comprimés, granules, pilules, suppositoires, lavements, ou suspensions capables d'être infusées par voie trans-endoscopique ou trans-colonoscopique dans le duodénum, l'iléum terminal ou via un tube entérique dans le jéjunum.
